(19) Europäisches Patentamt
European Patent Office
Office européen des brevets

(11) Veröffentlichungsnummer: **0 007 081**
**B2**

(12) # NEUE EUROPÄISCHE PATENTSCHRIFT

(45) Veröffentlichungstag der neuen Patentschrift: **05.11.86**

(21) Anmeldenummer: **79102315.3**

(22) Anmeldetag: **09.07.79**

(51) Int. Cl.⁴: **C 01 B 33/28,** B 01 J 29/28, C 07 C 1/20, C 07 C 2/12, C 07 C 2/66

(54) **Verfahren zur Herstellung von stickstoffhaltigen kristallinen Metallsilikaten mit Zeolithstruktur, nach dem Verfahren hergestellte Metallsilikate sowie deren Verwendung als Katalysatoren.**

(30) Priorität: **13.07.78 DE 2830787**

(43) Veröffentlichungstag der Anmeldung: **23.01.80 Patentblatt 80/2**

(45) Bekanntmachung des Hinweises auf die Patenterteilung: **11.11.81 Patentblatt 81/45**

(45) Bekanntmachung des Hinweises auf die Entscheidung über den Einspruch: **05.11.86 Patentblatt 86/45**

(84) Benannte Vertragsstaaten: **BE DE FR GB NL**

(56) Entgegenhaltungen:
**DE-A-2 442 240**
**DE-A-2 813 969**
**DE-A-2 817 575**
**DE-A-2 817 576**
**DE-A-2 817 577**
**DE-B-1 792 736**
**US-A-3 702 886**
**US-A-4 016 245**
**US-A-4 025 571**
**US-A-4 049 573**
**US-A-4 091 007**
**US-A-4 108 881**

(73) Patentinhaber: **BASF Aktiengesellschaft, Carl-Bosch- Strasse 38, D-6700 Ludwigshafen (DE)**

(72) Erfinder: **Marosi, Laszlo, Dr. Chem., Londoner Ring 11, D-6700 Ludwigshafen (DE)**
Erfinder: **Stabenow, Joachim, Dr. Phys., Am Feldrain 22, D-6940 Weinheim (DE)**
Erfinder: **Schwarzmann, Matthias, Dr. Chem., Carl-Bosch- Strasse 54, D-6703 Limburgerhof (DE)**

EP 0 007 081 B2

LIBER, STOCKHOLM 1986

**Beschreibung**

Die Erfindung betrifft ein Verfahren zur Herstellung von stickstoffhaltigen kristallinen Metallsilikaten mit Zeolithstruktur, die nach diesem Verfahren hergestellten neuen Metallsilikate, sowie deren erwendung als Katalysatoren.

Die gebräuchlichsten Zeolithe des A-, X- und Y-Typs haben eine große technische Bedeutung erlangt. Sie werden als Ionenaustauscher, Molekularsiebe und Katalysatoren technisch eingesetzt. Technische Verfahren wie das katalytische und hydrierende Kracken von Kohlenwasserstoffen werden mit Zeolithkatalysatoren durchgeführt. Neuerdings gewinnen Zeolithe vom Typ ZSM-5 oder ZSM-8 zunehmend an Interesse, mit denen neuartige Reaktionen, wie z.B. die Umwandlung von Methanol in flüssige Kohlenwasserstoffe katalysiert werden können. Die bekannten Zeolithkatalysatoren dieser Art weisen jedoch einige Nachteile auf, die ihre Anwendbarkeit bei den genannten Verfahren beeinträchtigen. Diese Nachteile bestehen in erster Linie in der aufwendigen Herstellung dieser Zeolithe. Ihre Herstellung erfolgt in Gegenwart von Natrium mit Hilfe von quaternären organischen Stickstoffbasen oder von organischen Aminen, z.B. n-Propylamin. Darüber hinaus können die primär bei der Synthese anfallenden alkalihaltigen Zeolithe noch nicht als Katalysatoren eingesetzt werden. Dazu ist es notwendig, die Zeolithe in die alkalifreie, saure H-Form überzuführen. Dieser Vorgang umfaßt in der Regel einen mehrstufigen Ionenaustausch, der oft auch mehrere Zwischenkalzinierungen erforderlich macht. Beim Ionenaustausch werden die Alkalikationen gegen Ammoniumionen ausgetauscht und daraus durch thermische Zersetzung die katalytisch aktive Wasserstofform hergestellt. Der häufige Ionenaustausch mit $NH_4$-Salzen, der in der Praxis mit großem Ammoniumüberschuß durchgeführt wird, verursacht zudem Umweltprobleme. Es ist deshalb wünschenswert, die Zeolithe gleich in der katalytisch aktiven, alkalifreien Form herzustellen, um dadurch die genannten Nachteile zu beseitigen.

Es ist jedoch bis heute nicht gelungen, katalytisch aktive Zeolithe ohne Alkali- oder Erdalkalikationen zu synthetisieren. Aus der Literatur ist lediglich eine einzige Zeolithsorte bekannt, die alkalifrei synthetisiert werden konnte (Helv. Chim. Acta 53, 1285 (1970)). Dieser Na-freie Gismodit besitzt jedoch keinerlei katalytische Bedeutung.

Bei allen anderen Zeolithsorten, die in Gegenwart von organischen stickstoffhaltigen Kationen hergestellt worden sind, wird ausdrücklich auf die erforderliche Anwesenheit von Alkalikationen während der Synthese hingewiesen und auch der hergestellte, Zeolith enthält signifikante Mengen an Alkaliionen, die durch Ionenaustausch aus dem Zeolithen entfernt werden müssen.

Die synthetischen kristallinen stickstoffhaltigen Molekularsiebe vom Typ A, X und Y können. beispielsweise ohne Na-Ionen nicht synthetisiert werden (Breck, D.W., Zeolite Molecular Sieves, 1974, S. 308) und auch der hergestellte Zeolith weist einen hohen Na-Gehalt auf. Die synthetischen kristallinen stickstoffhaltigen Molekularsiebe ZSM-5 und ZSM-8 müssen in der Synthesemischung einen Na-Gehalt, mindestens äquivalent zu dem eingesetzten Aluminium enthalten und auch der hergestellte Zeolith weist einen hohen Na-Gehalt auf.

Ein gemeinsames Merkmal der oben angeführten Zeolithsorten ist es, daß sie bei weitgehend gleicher Kristallgerüststruktur doch eigene Individuen darstellen. Die Zeolithe ZSM-5 und ZSM-8 unterscheiden sich beispielsweise in ihrem Kationenhaushalt in einer Weise, daß sie durch Ionenaustausch nicht ineinander überführbar sind. Darüber hinaus unterscheiden sich die stickstoffhaltigen Molekularsiebe vom A-Typ auch durch das Si/Al-Verhältnis in ihrem Kristallgerüst und auch durch ihre unterschiedliche katalytische Aktivität.

Ein weiteres gemeinsames Merkmal der bisher bekannten Zeolithsorten ist außerdem, daß sie nahezu ausschließlich kristalline Aluminosilikate sind. Ihre Zusammensetzung läßt sich aus einem Polysilikat $[SiO_{4/2}]_\circ$ ableiten. Ersetzt man einen Teil der Si-Atome durch Al-Atome, werden die fehlenden Valenzelektronen durch Einbau von Kationen nachgeliefert. Diese Kationen müssen nach dem heutigen Stand unserer Kenntnisse mindestens teilweise Alkali- oder Erdalkaliionen sein. Die Gerüstsubstitutionen, d.h. der isomorphe Ersatz von Al und/oder Si durch andere Elemente, ergibt heute nur eine sehr beschränkte Möglichkeit, Zeolithe mit neuartigen Eigenschaften herzustellen.

Der vorliegenden Erfindung liegt die Aufgabe zugrunde, aus billigen Ausgangsmaterialien neue, katalytisch aktive Zeolithe zu synthetisieren, die in Na-freier Form entstehen und daher ohne vorangehenden Ionenaustausch allenfalls nach einer einfachen Kalzinierung direkt als Katalysator eingesetzt werden können.

Es wurde gefunden, daß man neue stickstoffhaltige kristalline Metallsilikate mit Zeolithstruktur, die diese Bedingungen erfüllen, aus Mischungen von Siliziumdioxid und Metalloxiden und/oder Metallhydroxiden erhält, wenn man die Kristallisation in Abwesenheit von Alkali in wäßrigen Lösungen von Hexamethylendiamin vornimmt.

Es ist überraschend, daß unter den erfindungsgemäßen Kristallisationsbedingungen eine ganze eihe neuartiger Metallsilikatzeolithe darstellbar sind, auch solche die kein Aluminium mehr enthalten. An Stelle von Aluminium können verschiedene dreiwertige Metalle eingesetzt werden, z.B. Bor, Arsen, Antimon, Vanadium, Eisen oder Chrom. Im Falle der Aluminium-, Bor- und Eisensilikatzeolithe konnte nachgewiesen werden, daß diese Elemente überwiegend in tetraedrische Gitterplätze des Kristallgerüstes eingebaut sind. Die Elemente Chrom, Vanadium und Arsen sind wahrscheinlich nur zu einem Teil in das Kristallgerüst eingebaut, zum Teil sind sie in den intrakristallinen Poren eingelagert und besitzen keine definierten Gitterplätze. Die vorliegende Erfindung eröffnet die Möglichkeit, die einzigartigen Eigenschaften zeolthischer Aluminosilikate auch auf andere Metallsilikate zu übertragen und dadurch die Anwendungsmöglichkeiten von Zeolithen beträchtlich zu erweitern.

Eine bevorzugte Ausführungsform zur Synthese der neuen Na-freien, stickstoffhaltigen Zeolithe besteht

darin, daß man eine Reaktionsmischung aus $SiO_2$ und Metalloxid bzw. -hydroxid oder deren natriumfreiem Vorläufer in einer wäßrigen Hexamethylendiaminlösung 1 bis 8 Tage auf Temperaturen, zwischen 100 und 200° C, vorzugsweise 2 bis 4 Tage auf 140 bis 160° C unter dem Eigendruck erhitzt. Die neuen Verbindungen mit Zeolithstruktur besitzen eine dem Zeolithen ZSM-5 und ZSM-8 ähnliche Struktur, unterscheiden sich jedoch von diesen insbesondere in ihrer Zusammensetzung. Sie enthalten erfindungsgemäß kein Alkali und werden auch in Abwesenheit von Alkali-Ionen synthetisiert. Alkalifrei im Sinne der Erfindung bedeutet im wesentlichen frei von Natrium-Ionen. Der Restalkali-Gehalt solcher Zeolithe wird grundsätzlich nur durch Verunreinigungen der als Einsatzstoffe verwendeten Chemikalien verursacht. Es ist bekannt, daß technische Chemikalien immer Spuren von Natrium enthalten. So enthält käufliche pyrogene Kieselsäure (Aerosil), die besonders gut als Ausgangsmaterial geeignet ist, etwa 4 ppm $Na_2O$. Auch die zur Herstellung erforderlichen reaktiven Metalloxide bzw -hydroxide weisen oft einen unerwünschten Na-Gehalt auf. Es ist möglich, daß Na in Spuren die Kristallisation auch beschleunigen kann. Es sei aber ausdrücklich darauf hingewiesen daß in der Herstellung der erfindungsgemäßen Zeolithe grundsätzlich Na-arme Chemikalien bevorzugt werden, damit weitgehend Na-freie Zeolithe entstehen, die ohne nachfolgenden Ionenaustausch als Katalysatoren eingesetzt werden können. Vorhandene Na-Ionen wirken sich zwar nicht nachteilig auf die Synthese aus, machen aber u.U. einen nachfolgenden aufwendigen Kationenaustausch erforderlich. Die gleichen Einschränkungen müssen auch hinsichtlich des Aluminiumgehaltes der verwendeten Chemikalien gemacht werden. Neben diesem wesentlichen Unterschied der Alkali- und gegebenenfalls Aluminiumfreiheit sind auch die Kationen, die die negativen Ladungen der $[MeO_{4/2}]$ -Tetraeder kompensieren, je nach Amin verschieden und durch Ionenaustausch nicht beliebig ineinander überführbar. " Die erfindungsgemäß hergestellten Zeolithe sind als Katalysatoren zur Umwandlung von Kohlenwasserstoffen, z.B. Crack- und Hydrocrackverfahren, Oligomerisierung von für die Umsetzung von Methanol und/oder Dimethyläther zu ungesättigten Kohlenwasserstoffen durch Spaltung bei erhöhter Temperatur geeignet."

Sie werden hergestellt, indem man eine Mischung, die eine wäßrige Aminlösung, ein Metalloxid und/oder -hydroxid bzw. deren alkalifreie Vorläufer und Kieselsäure enthält, bei Temperaturen zwischen 100 bis 200° C zur Umsetzung bringt. ls Ausgangsmaterialien zur Herstellung der Zeolithe eignen sich insbesondere Metallnitrate, -sulfate, -oxide, -hydroxide una Oxyhydrate aber auch andere das reaktive Metalloxid liefernde Verbindungen. Als $SiO_2$-Quelle wird vorteilhaft pyrogene Kieselsäure (Aerosil) oder $SiO_2$-Sol eingesetzt, es können aber auch andere reaktive $SiO_2$-Sorten zur Anwendung kommen. Geeignet sind außerdem reaktive Metallsilikatgele, die beispielsweise durch Fällung aus Wasserglas und Metallsalz und Alkalifreiwaschen des Niederschlages hergestellt werden können. Die Konzentration der Hexamethylendiaminlösung kann zwischen 5 bis 90, vorteilhaft zwischen 20 bis 75 %, variieren. Besonders geeignet ist eine 50 %ige Lösung. Die Ausgangsmaterialien für die Metalloxide und $SiO_2$ werden in die Hexamethylendiaminlösung eingegeben und durch Rühren homogenisiert. Das homogene Gel wird dann in einem Autoklaven so lange unter Eigendruck erhitzt, bis ein kristallines Produkt gebildet wird, z.B. 2 bis 5 Tage bei 150° C.

Die so hergestellten Metallsilikatzeolithe enthalten in der Regel größere Mengen des verwendeten Hexamethylendiamins in den intrakristallinen Poren eingelagert. Das Amin kann beispielsweise durch Verbrennen kalzinieren aus den Poren entfernt werden, wobei katalytisch aktive Materialien entstehen.

Ein großer Vorteil des erfindungsgemäßen Verfahrens besteht darin, daß man z.B. bei Verwendung der Metalloxide bzw. -hydroxide die Mutterlauge im vollen Umfang wieder zur Herstellung von neuen Zeolithen einsetzen kann. Dies wird insbesnndere durch die Na-freie Herstellung erreicht.

Die Durchführung des erfindungsgemäßen Verfahrens wird an Hand der nachstehenden Beispiele näher erläutert.

Die in den Tabellen 1 bis 4 angegebenen Beugungsdiagramme wurden mit einem automatischen Phillips-Diffraktometer APD-10 hergestellt. Es wurde Kupferstrahlung zusammen mit einem Graphitmonochromator verwendet.

Die angegebenen Analysenwerte beziehen sich auf Trockenbasis. Die Substanzen wurden vor der chemischen Analyse so lange bei 550° C kalziniert, bis die eingeschlossenen Amine verbrannt waren. Die Differenz zu 100 % ergibt sich durch geringe Mengen adsorbiertes Wasser.

**Beispiel 1**

In 2000 g 25 %ige Hexamethylendiaminlösung wird bei 50°C 160 g Aerosil und frisch gefälltes $Al(OH)_3$ eingetragen. Das $Al(OH)_3$ wird aus 61,4 g $Al(NO_3)_3 \cdot 9\ H_2O$ durch Fällen mit Ammoniak hergestellt. Das $SiO_2/Al_2O_3$-Molverhältnis in der Mischung beträgt 32,6. Die Mischung wird dann so lange gerührt bis sie homogen ist und anschließend in einem Stahlautoklaven 5 Tage auf 150° C unter ihrem Eigendruck erhitzt. Das kristalline Produkt wird filtriert, gewaschen und bei 100° C getrocknet. Laut Röntgenanalyse besteht es aus gut kristallisiertem Aluminiumzeolith. Die chemische Analyse ergibt einen Na-Gehalt von 0,004 Gew.%. Die Analyse der Mutterlauge ergibt folgende Werte: 0,5 ppm $Na_2O$, 0,02 ppm $SiO_2$ und 1 ppm $Al_2O_3$. Die Ausbeute an Aluminiumzeolith, bezogen auf das eingesetzte $Al_2O_3$ und $SiO_2$, ist nahezu quantitativ.

**Beispiel 2**

Im Unterschied zu Beispiel 1 wird einmal die gleiche Gewichtsmenge einer 50 %igen Hexamethylendiaminlösung und einmal eine 70 %ige Hexamethylendiaminlösung verwendet. Die übrigen Reaktionsbedingungen werden konstant gehalten. Die Versuche ergeben ebenfalls gut kristallisierten Aluminiumzeolith.

**Beispiel 3**

In 2 Versuchen werden je 2000 g einer 50 %igen Hexamethylendiaminlösung eingesetz Mengen der übrigen Reaktanten sind die gleichen wie in Beispiel 1. Die Reaktionsdauer betragt 2,5 bzw. 3 Tage bei 1 50°C. Das Reaktionsprodukt ist in beiden Fällen kristallin und zeigt nach Kalzinie bei 550°C ein für den Aluminiumzeolith charakteristisches Röntgenbeugungsdiagramm mit folgenden signifikaten d-Werten:

**TABELLE 1**

| Netzebenenabstände d (Å) | Relative Intensität (I/Io) |
|---|---|
| 11,02 | 83 |
| 9,90 | 64 |
| 5,94 | 18 |
| 5,54 | 13 |
| 4,98 | 8 |
| 4,35 | 7 |
| 3,83 | 100 |
| 3,70 | 43 |
| 3,62 | 21 |
| 3,32 | 9 |
| 3,30 | 7 |
| 3,04 | 10 |
| 2,97 | 11 |
| 2,60 | 5 |
| 2,48 | 5 |
| 2,39 | 4 |
| 2,00 | 10 |
| 1,98 | 9 |

**Beispiel 4**

Bei diesem Versuch werden anstelle der ursprünglichen Hexamethylendiaminlösung 1 650 g Mutterlauge eingesetzt, die aus Versuchen mit 50 %iger Hexamethylendiaminlösung isoliert wurde.Die Reaktionsdauer beträgt 4 Tage, die übrigen Parameter entsprechen den Angaben im Beispiel 1. Man erhält wiederum gut kristallisierten Aluminiumzeolith. Die Muterlauge hat einen $Na_2O$-Gehalt von 0,5 ppm, das eingesetzte Aerosil hat einen $Na_2O$-Gehalt von etwa 4 ppm.

**Beispiel 5**

In 2000 g 50 %ige Hexamethylendiaminlösung werden bei 60°C 160 g Aerosil und frisch gefälltes $Al(OH)_3$ eingetragen. Das $Al(OH)_3$ wird aus 30,7 g $Al(NO_3)_3.9\ H_2O$ durch Fällen mit Ammoniak hergestellt. Das $SiO_2/Al_2O_3$-Molverhältnis in der Mischung beträgt etwa 100. Die Mischung wird so lange gerührt bis sie homogen ist und anschließend in einem Stahlautoklaven 5 Tage auf 150°C unter ihrem Eigendruck erhitzt. Das kristalline Produkt wird filtriert, gewaschen und bei 100°C getrocknet. Laut Röntgenanalyse besteht es aus gut kristallisiertem Aluminiumzeolith. Das erhaltene Produkt wird 10 Stunden bei 550°C kalziniert und anschliessend analysiert. Die chemische Analyse ergibt folgendes Ergebnis:

0,002 % $Na_2O$, 1,53 % $Al_2O_3$, 96,3 % $SiO_2$.

**Beispiel 6**

Im Gegensatz zu den vorangehenden Beispielen wird an Stelle von Aluminiumhydroxid 110 g $Al_2(SO_4)_3.18H_2O$ eingesetzt. 150 g Aerosil werden in 2000 g 50 %ige Hexamethylendiaminlösung eingerührt und anschließend eine gesättigte wäßrige Lösung von 110 g $Al_2(SO_4)_3.18\ H_2O$ hinzugegeben. Die erhaltene Mischung wird 4 Tage auf 160°C erhitzt, anschließend filtriert und gewaschen. Das Produkt besteht auch in diesem Fall aus gut kristallisiertem Aluminiumzeolith.

**Beispiel 7**

Diese Beispiel beschreibt die Herstellung eines kristallinen Na-freien Borosilikats. In 1 700 g 50 %ige Hexamethylendiaminlösung werden 160 Aerosil eingerührt und zu dieser Suspension eine Lösung von 10,1 g Borsäure in 300 g 50 %iger Hexamethylendiaminlösung bei 60°C hinzugegeben. Die erhaltene Mischung wird etwa 15 Minuten durch Rühren homogenisiert und anschließend in einem Stahlautoklaven 5 Tage auf 150°C unter ihrem Eigendruck erhitzt. Das kristalline Produkt wird filtriert, gewaschen und bei 100°C getrocknet. Es besteht aus gut kristallisiertem Borzeolith.

Ein Teil der Kristalle wird etwa 10 Stunden bei 550°C kalziniert. Die chemische Analyse ergibt folgendes Ergebnis:

0,0035 % $Na_2O$, 0,73 % $B_2O_3$, 91,5% $SiO_2$

Molverhältnis $SiO_2/B_2O_3$ = 145

Die Röntgenbeugungsanalyse ergibt nach der Kalzinierung bei 550°C folgende signifikante d-Werte.

**Tabelle 2**

| Beispiel 7 | | Beispiel 8 b | |
|---|---|---|---|
| Netzebenenabstände d (Å) | Relative Intensität (I/Io) | Netzebenenabstände d (Å) | Relative Intensität (I/Io) |
| 10,95 | 67 | 10,96 | 55 |
| 9,85 | 59 | 9,77 | 43 |
| 5,93 | 19 | 5,91 | 12 |
| 5,53 | 10 | 5,51 | 9 |
| 4,98 | 9 | 4,33 | 9 |
| 3,81 | 100 | 3,80 | 100 |
| 3,69 | 32 | 3,69 | 43 |
| 3,62 | 21 | 3,63 | 19 |
| 3,03 | 7 | 3,30 | 10 |
| 2,96 | 13 | 3,02 | 8 |
| 1,995 | 10 | 2,95 | 12 |
| 1,989 | 8 | 1,985 | 10 |
| | | 1,979 | 9 |

**Beispiel 8**

In 2 Versuchen wird eine 50 %ige Hexamethylendiaminlösung eingesetzt. In Versuch a) werden 2,5 g, in Versuch b) 20,2 g Borsäure verwendet. Die übrigen Reaktionsbedingungen werden wie in Beispiel 7 gehalten. Das Reaktionsprodukt ist in beiden Fällen kristallin. Probe 8b zeigt nach Kalzinierung bei 550°C ein Röntgenbeugungsdiagramm mit den d-Werten wie sie in Tabelle 2 angegeben sind.

**Beispiel 9**

Diese Beispiel beschreibt die Herstellung eines kristallinen Na-freien Arsensilikats.

In 88 g 50 %ige Hexamethylendiaminlösung werden 11,45 g Aerosil bei 70°C eingerührt und zu dieser Suspension eine Lösung von 2,32 g $As_2O_3$ in 30 g 50%iger Hexamethylendiaminlösung hinzugegeben. Die erhaltene Mischung wird 5 Tage auf 150°C unter ihrem Eigendruck erhitzt. Das erhaltene Produkt wird filtriert, gewaschen und bei 100°C getrocknet. Es besteht aus gut kristallisiertem Arsenzeolith.

Die chemische Analyse des kalzinierten Produktes ergibt folgendes Ergebnis:

0,008% $Na_2O$, 0,25 % $As_2O_3$, 96,0 % $SiO_2$

Das Ergebnis der Röntgenbeugungsanalyse ist in der Tabelle 3 zusammengefaßt:

**TABELLE 3**

| Netzebenenabstände d (Å) | Relative Intensitäten (I/Io) |
|---|---|
| 11,12 | 52 |
| 9,99 | 73 |
| 9,97 | 6 |
| 6,70 | 5 |
| 6,36 | 6 |
| 5,97 | 21 |
| 5,69 | 6 |
| 5,56 | 9 |
| 5,01 | 9 |
| 4,60 | 3 |
| 3,846 | 100 |
| 3,818 | 62 |
| 3,744 | 17 |
| 3,712 | 21 |
| 3,620 | 20 |

**Beispiel 10**

Dieses Beispiel beschreibt die Herstellung eines kristallinen Na-freien Antimonsilikates.

In 88 g 50 %ige Hexamethylendiaminlösung werden 11,45 g Aerosil bei 60°C eingerührt und zu dieser Mischung eine Suspension von 3,42 g $Sb_2O_3$ in 30 g 50 %iger Hexamethylendiaminlösung hinzugegeben. Die erhaltene homogene Mischung wird 5 Tage auf 150°C unter ihrem Eigendruck erhitzt. Das erhaltene Produkt wird filtriert, gewaschen und bei 100°C getrocknet.

Das Beugungsdiagramm des Produktes zeigt neben $Sb_2O_3$ eine weitere kristalline Komponente, deren Beugungslinien im wesentlichen den in Tabelle 1 angegebenen Linien entsprechen.

**Beispiel 11**

Dieses Beispiel zeigt, daß unter den erfindungsgemäßen Reaktionsbedingungen Mischungen von Hexamethylendiamin, Wasser und Aerosil ohne Zusatz von Al-, Boder As-Oxiden oder Sb nicht zu den beschriebenen kristallinen Verbindungen kristallisieren.

In 236 g 50 %ige Hexamethylendiaminlösung werden 22,9 g Aerosil eingetragen und die Mischung durch Rühren homogenisiert. Die erhaltene Mischung wird in zwei Teile geteilt und in Stahlautoklaven 5 Tage auf 150°C bzw. 175°C unter Eigendruck erhitzt. Die erhaltenen Reaktionsprodukte sind gelig, in ihren Beugungsdiagrammen werden keine Beugungslinien der oben beschriebenen Verbindungen festgestellt.

**Beispiel 12**

100 g des in Beispiel 7 erhaltenen Borzeolith werden mit Böhmit au 1 mm-Strängen verarbeitet und die Stränge 10 Stunden bei 550°C kalziniert. Der Zeolithgehalt der Stränge beträgt 65 Gew.%, 20 g dieses

Katalysators werden dann in einen Durchflußreaktor eingebaut und die Aktivität in verschiedenen Reaktionen gestestet:

a) Olefinoligomerisierung

Bei 380°C wird Propylen mit einer Belastung von 40 g/h über den Katalysator geleitet. Die Umsetzung beträgt etwa 50 %. Das erhaltene flüssige Reaktionsprodukt hat folgende Zusammensetzung:

13% Aromaten
20% Aliphaten
67% $C_5$-$C_9$-Olefine

b) Umsetzung von Methanol

Rohrmethanol mit einem Wassergehalt von 17 Gew.% wird bei 370°C über einen Aluminiumoxidkatalysator dehydratieiert und die erhaltene Gesmichsung bei 370°C über den Zeolithkatalysator umsetzt. Die Belastung beträgt 46 g Dimethylläther/h. Die Reaktionsbedingungen sind in der folgenden Tabelle zuzammengefaßt:

Eingangstemperatur: 370°C
Druck:1,14 bar
Temperaturansteig: 70°C
Umsetzung: 95-100%
Gesamtbelastung, g Dimethyläther: 120g.

Das erhaltene Reaktionsprodukt hat folgende Zusammenstezung:

25 Gew.% Öl, bezogen auf eingesetztes $CH_2$, bestehend aus 40% Aromaten, 13% Aliphaten, 47% $C_5$-$C_9$-Polyolefine.

Gasförmige Reaktionsprodukte: 75 Gew.%, Bezogen auf eingesetztes $CH_2$, 8,6 Gew.% Äthylen, 58 Gew.% Propylen, 17,5 Gew.% Butene, 2,75 Gew.% Methan, 3,75 Gew.% Propan und 9 Gew.% Butene.

**Beispiel 13**

100 g des in Beispiel 2 erhalten Zeoliths werden mit Böhmit zu 1 mm-Strängen verarbeitet und die Stränge 10 Stunden bei 550°C kalziniert. Der Zeolithgehalt der Stränge beträgt 65 Gew.%.

18 g dieses Katalysators werden in einen Durchflußreaktor eingebaut und

a) die Aktivität be der Umsetzung von Methanol in Olofine gestestet.

Die Reaktionsbedingungen und die Versuchsergebnisse sind in der nachfolgenden Tabelle dargestellt:

Eingangstemperatur 370°
Temperaturanstieg 100°C
Druck 1,2 bar

| | | |
|---|---|---|
| Beschickung | $CH_3OH$ (g) | 62 |
| | $H_2O$ (g) | 180 |

Belastung (Beschickung/h) 400 g
Gesamtebelastung, g Methanol 730 g
Umsetzung 95-100%

Man erhält ein Reaktionsprodukt mit folgender Zusammensetzung:

Öl 22g
Gasförmige Kohlenwasserstoff (Vol.%)
Olefine $C_2$ 30
$C_3$ 32
$C_4$ 17
Paraffine $C_1$ 2
$C_2$ Spuren
$C_3$ 3
$C_4$ 13

b) Alkylierung von Benzol mit Äthylen

Reaktionstemperatur: 400°C
Druck: 1 bar
Belastung: 16 g Äthylen/h
312 g Benzol/h

Zusammensetzung der Reaktionsprodukte
17 Gew.% Äthylbenzol
1,5 Gew.% Diäthylbenzol
0,1 Gew.% Toluol
81,5 Gew.% Benzol.

Die Reaktionsdauer beträgt etwa 60 Stunden. Die Selektivität liegt in diesem Zeitraum bei 98 bis 100 %, die Ä

thylenkonversion beträgt >95%.

**Patentansprüche**

1. Verfahren zur Herstellung von stickstoffhaltigen kristallinen silikaten dreiwertiger Metalle mit Zeolithstruktur aus Siliziumdioxid und Metalloxiden und/oder Metallhydroxiden, dadurch gekennzeichnet, daß man die Kristallisation in Abwesenheit von Alkali in einer wäßrigen Lösung von Hexamethylendiamin vornimmt.

2. Verfahren nach Anspruch 1, dadurch gekennzeichnet, daß man die Kristallisation unter dem Eigendruck der Lösungen bei Temperaturen von 100 bis 200° C durchführt.

3. Verfahren nach Anspruch 1, dadurch gekennzeichnet, daß man eine 20 bis 75 %ige Hexamethylendiaminlösung verwendet.

4. Aluminiumsilikatzeolithe, hergestellt nach Anspruch 1 bis 3.

5. Borosilikatzeolithe, hergestellt nach Anspruch 1 bis 3.

**Claims**

1. A process for the manufacture of nitrogen-containing crystalline silicates of trivalent metals, said silicates having a zeolite structure, from silicon dioxide and metal oxides and/or metal hydroxides, characterized in that the crystallization is carried out in the absence of an alkali metal, in an aqueous solution of hexamethylenediamine.

2. A process as claimed in claim 1, characterized in that the crystallization is carried out under the autogenous pressure of the solution, at from 100° to 200° C.

3. A process as claimed in claim 1, characterized in that a hexamethylenediamine solution of from 20 to 75% strength is used.

4. An aluminum silicate zeolite manufactured as claimed in claims 1 to 3.

5. A borosilicate zeolite manufactured as claimed in claims 1 to 3.

**Revendications**

1. Procedé pour la préparation de silicates de métaux trivalents cristallins azotés à structure zéolitique à partir de bioxyde de sailicium et dioxydes métalliques et/ou d'hydroxydes métalliques, caractérisé en ce qu'on procède à la criatallisation en l'absence d'alcali dans une solution aqueuse d'hexaméthylènediamine.

2. Procédé selon la revendication 1, caractérisé en ce qu'on effectue la cristallisation sous la pression propre des solutions à dea températures de 100 à 200° C.

3. Procédé selon la revendication 1, caractérisé en ce qu'on utilise une solution d'hexaméthylènediamine dont la concentration se situe entre 20 et 75 %.

4. Zéolites de ailicate d'aluminium, préparées selon l'une quelconqua des revendications 1 à 3.

5. Zéolites de silicate de bore, préparéas selon l'une quelconque dea revendications 1 à 3.